# EUROPEAN PATENT SPECIFICATION

(11) **EP 0 518 448 B1**
(45) Date of publication and mention of the grant of the patent: **24.09.1997**
(21) Application number: 92201728.0
(22) Date of filing: 12.06.1992
(51) Int. Cl.: C07K 16/04, A23J 1/20

(54) **A method for isolating proteins from milk and a method for processing milk**
Verfahren zur Isolierung von Proteinen aus Milch und Verfahren zur Behandlung von Milch
Méthode pour isoler des protéines du lait et méthode pour traiter le lait

(30) Priority: 14.06.1991 NL 9101032
(43) Date of publication of application: 16.12.1992
(73) Proprietor: N.V. JOYVALLE, B-9320 Erembodegem-Aalst (BE)
(72) Inventor: Vervaeck, Jan, B-9630 Zwalm (BE); Decatelle, Johan, B-9840 De Pinte (BE); van Coillie, Yves, B-8830 Hooglede-Gits (BE)
(74) Representative: De Hoop, Eric

(56) References cited:
- WO-A-90/12803
- BE-A- 901 672

## Description

The invention relates to the isolation of the major antimicrobial proteins from milk, in particular a mixture consisting of immunoglobulins (Ig), lactoferrin (LF) and lactoperoxidase (LP).

Immunoglobulins, lactoferrin and lactoperoxidase are proteins having antimicrobial and antiviral activity. They are present in milk in low concentrations, i.e. 0.5, 0.2 and 0.05 g/l, respectively.

The antimicrobial effect of these components has been proven more than once: immunoglobulins affect the immune system; lactoferrin complexes iron as a result of which iron is no longer available for micro-organisms; lactoperoxidase is an enzyme which forms from hydrogen peroxide and SCN-(thiocyanate) a micro-organism inhibiting substance (OSCN-, hypothio-cyanate).

The isolation of lactoferrin and lactoperoxidase from milk and/or whey is described in Dutch patent application 86 00231. US patent specification 4,791,193 only discloses the isolation of lactoferrin from milk and/or whey.

French patent application 2,452,881 discloses the isolation of milk proteins from milk and/or whey, particularly the isolation of α-lactalbumin and β-lactoglobulin.

The first two methods mentioned above use cation exchange. The last mentioned method uses both cation and anion exchange and exclusion chromatography.

Lactoferrin and lactoperoxidase have an iso-electric point pI>8, so that cation exchange is obvious at neutral or acidic pH. This results into a high purity, since all other milk proteins, which are present in a considerable concentration, have a pI of about pH 5. In the first two methods mentioned above it is possible to isolate lactoferrin and lactoperoxidase without adjusting the pH of the raw material.

The isolation of immunoglobulins using ion exchange requires a pH adjustment of the raw material. Immunoglobulins have iso-electric points varying from pH 5.8 to 8.3. For efficient isolation by means of anion exchange the pH should be higher than 9 and by means of cation exchange the pH should be lower than 5.

Consequently, if ion exchange is used, a pH adjustment of the raw material is required for simultaneously isolating the three proteins mentioned above. If the raw material is milk, the pH should be brought below 5 for cation exchange, which causes coagulation of the caseins. Anion exchange is also not quite efficient: at a pH higher than 9 lactoferrin and lactoperoxidase have a positive or neutral charge, so that they will not be isolated.

The present invention provides a method for simultaneously isolating immunoglobulins, lactoferrin and lactoperoxidase from skimmed, non-sterilized milk in which these proteins are present in native form. The milk fraction obtained by this method, from which the mentioned proteins have been removed, in principle retains its original composition, except for the absence of the isolated proteins.

The present invention provides a method for isolating proteins from milk, characterized in that skimmed, non-sterilized milk is subjected to metal-chelate-interaction column chromatography, comprising the consecutive steps of:
a) contacting the milk with a transition metal which is chelated to a functional matrix having a mechanical strength and porosity such that for a column having a length of 30 cm and when using a linear flow rate of 150 cm/h, the pressure is in the range of 0-5 bar and is stable, as a result of which milk proteins are bonded to the matrix,
b) removing the fraction containing milk components not bonded by the matrix, and
c) desorbing the proteins bonded to the matrix.

The method used is metal-chelate-interaction-chromatography (MCIC) or metal-chelate-affinity-chromatography (MCAC). This form of chromatography uses the characteristic of transition metals to interact, in chelated form, with proteins containing at the surface one or more of the following amino acids: histidine, cysteine and tryptophane. Copper, nickel, zinc, cobalt and the like (alkali and alkaline earth metals) may be used as transition metal. The group chelating the metal is bonded to an inert carrier. As chelating groups may be used i.a. di- and tris-acetate groups (e.g. imino-diacetate, tris-carboxy-ethylene-diamine).

The inert carrier with the chelating groups bonded thereto is indicated as the functional matrix in the description and the claims. The principle of MCIC may be represented by the following:
INNERT CARRIER---CHELATING GROUP---METAL---PROTEIN MCIC has been used for isolating immunoglobulins and lactoferrin from whey (J. Dairy Science, 71, 1747-1755, 1988; Affinity Chromatogr. Biol. Recognit. 269-273, 1983) and lactoferrin from whey of human milk (FEBS letters, 75, 1, 89-92, 1977).

The use of milk as raw material for MCIC has not been proposed up till now. That is because on the one hand it is not attractive to use a valuable product such as milk for isolating only a few components therefrom. Whey, on the contrary, is in fact a waste product and isolating as many as possible components therefrom is commercially attractive. Whey originating from the preparation of cheese, however, usually has been subjected to a heat treatment, so that the isolated proteins are partially or entirely inactivated. In addition, when preparing cheese a part of the lactoperoxidase activity precipitates, so that the whey originating therefrom is already much poorer in lactoperoxidase.

On the other hand the use of milk as raw material for MCIC has not been proposed up till now as one is apprehensive of the column, in which the matrix is preferably used, to clog, since milk has a higher viscosity than whey and moreover contains casein micelles having a diameter of 30 to 300 nm. A higher counterpressure will appear as a result thereof when milk is applied to the chromatographic matrix. This pressure may compress the chromatographic matrix. If this compression becomes too intense the column will clog eventually. It appeared that e.g. by using Sepharose 6B, which is mentioned in J. Dairy Science loc. cit., the column will clog already after a short period at a linear flow rate of 150 cm/h. Even the cross-linked equivalent, Sepharose CL-6B, appeared to be unsuitable when using skimmed milk.

It has been found that particular matrices can be applied when using skimmed milk. The matrix should meet the following major requirements:
a high mechanical strength and a high porosity. For a column having a length of 30 cm and when using a linear flow rate of 150 cm/h, the pressure should be in the range of 0-5 bar and should be approximately stable during application of the sample. Examples of suitable functional matrices are: Chelating Sepharose Fast Flow (Pharmacia) and Amicon Cellufin Chelate (Amicon).

In carrying out the present method the raw material does not need to be conditioned, i.e. neither the acidity nor the ionic strength, nor any other physical-chemical parameter of the sample has to be adjusted. The bonding of the proteins concerned may be carried out at the natural pH and ionic strength of the milk. This is contrary to other chromatographic techniques such as ion exchange, in which the pH plays an important role, or hydrophobic interaction, in which ionic strength is very important. In addition, the fraction containing unbonded components (flow-through) can further be used as a valuable intermediate product, since pH and ionic strength have remained the same. Since the isolated proteins are unstable when heated and would be denatured during sterilization, the flow-through is substantially identical to ordinary sterilized milk or UHT-milk as far as active components are concerned.

However, it has appeared that the fraction containing unbonded components shows a considerably increased metal content. It has appeared that milk, and more in particular the casein micelles, absorbs the metals very easily. Tests have proved that this problem hardly occurs when using whey as raw material. In the flow-through of whey the metal content has barely increased, while when using milk as raw material, a metal concentration of 50 to 100 times higher is found in the flow-through, even when only the first half of the column is saturated with the metal (50% saturation); the metal released from this first half is then yet partially taken up in the second, unsaturated half. So as to enable further processing of this flow-through, it is absolutely necessary to remove this metal.

Now it has been found that the metals may be completely or substantially removed from the fraction containing unbonded components of the milk by contacting it at least once with a functional matrix as used in step a), only this time not charged with transition metal. In this manner the milk is depleted step by step in respect of the metals complexed on the casein micelles.

Therefore, according to another aspect of the invention a method is provided for processing milk, characterized in that skimmed, non sterilized milk is subjected to metal-chelate-interaction column chromatography, comprising the consecutive steps of:
a) contacting the milk with a transition metal which is chelated to a functional matrix having a mechanical strength and porosity such that for a column having a length of 30 cm and when using a linear flow rate of 150 cm/h, the pressure is in the range of 0-5 bar and is stable, as a result of which milk proteins are bonded to the matrix,
b) removing the fraction containing milk components not bonded by the matrix,
c) desorbing the proteins bonded to the matrix, and
d) contacting the fraction obtained in step b) at least once with a functional matrix as used in step a), only this time not charged with transition metal, whereby metal present in the fraction is completely or substantially removed.

It is preferred to contact the fraction obtained in step b) 3-7 times batchwise with each time a fresh functional matrix in step d).

The milk fraction containing unbonded components thus obtained in step d) is in principle suitable for consumption. However, it is paricularly advantageous and suitable to subject this milk fraction to a heat treatment, UHT or sterilization, and to add the isolated proteins thereto. In this manner a long-life milk is obtained wherein the mentioned heat-unstable proteins are present in native form.

The method according to the invention is described in detail hereinafter.

The method may by be carried out batchwise and/or preferably continuously (in a column). In practice, the method comprises the consecutive steps of:
1) Applying (chelating) the transition metal on the functional matrix. The degree of saturation (i.e. the gel volume saturated with the metal) is 20-100%.
2) Rinsing with water.
3) Equilibrating with a buffer system (e.g. phosphate buffer).
4) Applying the raw skimmed milk and obtaining a continuous eluate.
5) Rinsing with equilibration buffer for removing the unbonded components.
6) Desorbing the bonded components (Ig, LF, LP); this step may be effected in various manners:
   a) decreasing the pH (step by step or in gradient form): e.g. acetic acid/acetate buffer pH 3 + NaCl (0-1 M). Adding salt may prevent precipitation of the desorbed proteins,
   b) increasing the pH to a value of 8-10. This has the advantage that the casein micelles, which remained in the matrix, are broken down. Moreover immunoglobulins are more stable at pH 9 than for instance at pH of 3. Example: Tris-buffer + NaCl 0-1 M,
   c) desorbing with distilled water, so that most proteins are desorbed,
   d) competitive ligands (e.g. imidazole, histamine, glycine, ammoniumchloride, etc.) + NaCl 0-1 M,
   e) chelating components (e.g. EGTA, EDTA, etc.) + NaCl 0-1 M, or a combination of the above possibilities, e.g. first increasing the pH and then descreasing the pH,
7) In the case of desorption by means of possibility a, b, c or d: stripping the transition metal by means of chelating components (e.g. EGTA, EDTA, etc.) + NaCl 0-1 M.
8) Rinsing with water.
9) After treatment of the flow-through milk fraction by contacting it with a non-charged functional matrix.

In the desorption fraction both immunoglobulins, lactoferrin and lactoperoxidase are recovered in native form. In the initial application of the sample β-lactoglobulin, α-lactalbumine and albumine (BSA) are bonded too. However, these are substituted by immunoglobulins, lactoferrin and lactoperoxidase when further applying the sample.

The protein preparation isolated by the present method, which preparation particularly consists of immunoglobulins, lactoferrin and lactoperoxidase may be formulated to pharmaceutical compositions or may be added to a plurality of nutrients. As aforesaid, preferably the protein preparation is added to the above-mentioned flow-through milk from which the metals are completely or substantially removed and which is then sterilized.

### Example

A column having an internal diameter of 16 mm and a length of 15cm was filled with 30 ml of Chelating Sepharose Fast Flow (Pharmacia). Half of this column was saturated with copper: 124 mg of CuS0₄.5H₂O in 50 ml of distilled water. After rinsing with water and equilibration buffer (phosphate 0.05 M, pH 6.7) 960 ml of raw, skimmed milk was passed through the column at a linear flow rate of 150 cm/h (300 ml/h). The unbonded substances were eluated with equilibration buffer. The desorption was carried out by sequentially increasing the pH (Tris-HC1 0.1 M, pH 9) and decreasing the pH (acetic acid-acetate 0.1 M + NaCl 0.2 M, pH 3). Finally the metal was stripped from the column by means of EDTA 0.05 M + NaCl 0.5 M. After each run the metal should be stripped, because part of the copper has already been lost in the flow-through and the copper still bonded has come closer to the exit of the column, so that the loss of copper would be greater in a next run.

Totally 481 mg of protein was desorbed namely 315 mg of immunoglobulins, 74 mg of lactoferrin, 57 mg of lactoperoxidase, 15 mg of BSA and 20 mg of α-lactalbumin. Thus, the purity of antimicrobial substances (immuno-globulins, lactoferrin and lactoperoxidase) was 93%.

The content of copper in the flow-through was 4.6 ppm. This was removed by contacting this milk batchwise 5 times with uncharged Chelating Sepharose Fast Flow, in a milk/gel ratio of 25/1. Each contact took place at 8°C for 1 hour. The final copper content was 72 ppb.

The immunoglobulins, lactoferrin and lactoperoxidase isolated in this manner were after filter sterilization (filter membrane 0.2 µm) added aseptically to the flow-through having the reduced content of copper of 72 ppb after sterilization thereof, thereby obtaining a long-life milk having retained the activity of said heat-unstable components.

## Claims

1. A method for isolating proteins from milk, characterized in that skimmed, non-sterilized milk is subjected to metal chelate interaction column chromatography, comprising the consecutive steps of:
a) contacting the milk with a transition metal which is chelated to a functional matrix having a mechanical strength and porosity such that for a column having a length of 30 cm and when using a linear flow rate of 150 cm/h, the pressure is in the range of 0-5 bar and is stable, as a result of which milk proteins are bonded to the matrix,
b) removing the fraction containing milk components not bonded by the matrix, and
c) desorbing the proteins bonded to the matrix.

2. A method according to claim 1, **characterized in that** this method is carried out continuously in a column.

3. A method according to claim 1 or 2, **characterized in that** the transition metal is copper, nickel, zinc or cobalt.

4. A method according to any of claims 1-3, **characterized in that** in step c) the proteins are desorbed by means of distilled water or by increasing the pH to a value of 8-10, after which any transition metal present is removed by a chelated substance.

5. A method according to any of claims 1-4, **characterized in that** in step c) salt is added to a maximum of 1M during desorption.

6. A method according to any of claims 1-5, **characterized in that** the desorbed proteins mainly consist of immunoglobulins, lactoferrin and lactoperoxidase.

7. A method for processing milk, **characterized in that** skimmed. non-sterilized milk is subjected to metal chelate interaction column chromatography, comprising the consecutive steps of:
a) contacting the milk with a transition metal which is chelated to a functional matrix having a mechanical strength and porosity such that for a column having a length of 30 cm and when using a linear flow rate of 150 cm/h, the pressure is in the range of 0-5 bar and is stable, as a result of which milk proteins are bonded to the matrix,
b) removing the fraction containing milk components not bonded by the matrix,
c) desorbing the proteins bonded to the matrix, and
d) contacting the fraction obtained in step b) at least once with a functional matrix as used in step a), only this time not charged with transition metal, whereby metal present in the fraction is completely or substantially removed.

8. A method according to claim 7, **characterized in that** the fraction obtained in step b) is contacted 3-7 times batchwise with each time a fresh functional matrix in step d).

9. A method according to claim 7 or 8, **characterized in that** the fraction obtained in step d) is subsequently sterilized, whereafter the proteins obtained in step c) are added thereto.

## Patentansprüche

1. Verfahren zur Isolierung von Proteinen aus Milch, **dadurch gekennzeichnet**, dass man abgerahmte, nichtsterilisierte Milch einer Metal-Chelat-Interaktion-Säule-Chromatographie unterwirft, wobei aufeinanderfolgend
a) die Milch mit einem Übergangsmetall, das an einer funktioneller Matrix mit einer derartigen mechanischen Stärke und Porosität, dass für eine Säule mit einer Länge von 30 cm und bei einer linearen Strömungsgeschwindigkeit von 150 cm/S, der Druck im Bereich von 0-5 bar liegt und stabil ist, cheliert ist, in Kontakt gebracht wird so dass Milchproteine an die Matrix gebunden werden,
b) die Fraktion mit nicht von der Matrix gebundenen Milchkomponenten entfernt wird,
c) die an der Matrix gebundenen Proteine desorbiert werden.

2. Verfahren nach Anspruch 1, **dadurch gekennzeichnet**, dass das Verfahren ununterbrochen in einer Säule durchgeführt wird.

3. Verfahren nach Anspruch 1 oder 2, **dadurch gekennzeichnet**, dass das Übergangsmetall Kupfer, Nickel, Zink oder Kobalt ist.

4. Verfahren nach einem der Ansprüche 1 bis 3, **dadurch gekennzeichnet**, dass in Stufe c) die Proteine mittels distillierten Wassers oder mittels Erhöhung des pH's bis einen Wert von 8 bis 10 desorbiert werden, wonach jedes eventuell vorliegende Übergangsmetall mittels einer chelierten Substanz entfernt wird.

5. Verfahren nach einem der Ansprüche 1 bis 4, **dadurch gekennzeichnet**, dass in Stufe c) Salz bis ein Maximum von 1M während Desorption zugefügt wird.

6. Verfahren nach einem der Ansprüche 1 bis 5, **dadurch gekennzeichnet**, dass die desorbierten Proteine hauptsächlich aus Immunoglobulinen, Laktoferrin und Laktoperoxidase bestehen.

7. Verfahren für die Verarbeitung von Milch, **dadurch gekennzeichnet**, dass abgerahmte, nicht sterilisierte Milch einer Metal-Chelat-Interaktion-Säule-Chromatographie unterworfen wird, wobei aufeinanderfolgend
a) die Milch mit einem Übergangsmetall, das an einer funktioneller Matrix mit einer derartigen mechanischen Stärke und Porosität, dass für eine Säule mit einer Länge von 30 cm und bei einer linearen Strömungsgeschwindigkeit von 150 cm/S, der Druck im Bereich von 0-5 bar liegt und stabil ist, cheliert ist, in Kontakt gebracht wird, so dass Milchproteine an die Matrix gebunden werden,
b) die Fraktion mit nicht von der Matrix gebundenen Milchkomponenten entfernt wird,
c) die an der Matrix gebundenen Proteine desorbiert werden.
d) die in Stufe b) erhaltene Fraktion mindestens einmal mit einer wie im Stufe a) angewandten funktioneller Matrix in Kontakt gebracht wird, aber dieses Mal nicht mit Übergangsmetall geladen, wobei das in der Fraktion vorliegende Metall vollständig oder nahezu vollständig entfernt wird.

8. Verfahren nach Anspruch 7, **dadurch gekennzeichnet**, dass die in Stufe b) erhaltene Fraktion 3 bis 7mal ladungweise mit jeweils einer frischen funktionellen Matrix in Stufe d) in Kontakt gebracht wird.

9. Verfahren nach Anspruch 7 oder 8, **dadurch gekennzeichnet,** dass die in Stufe d) erhaltene Fraktion dann sterilisiert wird, wonach die in Stufe c) erhaltenen Proteine daran zugefügt werden.

## Revendications

1. Procédé pour isoler les protéines du lait, caractérisé en ce qu'on soumet du lait non stérilisé écrémé à une chromatographie sur colonne avec interaction de chélate métallique, comprenant les étapes consécutives de:
a) mise en contact du lait avec un métal de transition qui est chélaté à une matrice fonctionnelle ayant une résistance mécanique et une porosité telles que pour une colonne ayant une longueur de 30 cm et lorsqu'on utilise un débit linéaire de 150 cm/h, la pression est dans un intervalle de 0-5 bar et est stable, à la suite de quoi les protéines du lait sont liées à la matrice,
b) enlèvement de la fraction contenant les composants du lait non liés par la matrice, et
c) désorption des protéines liées à la matrice.

2. Procédé selon la revendication 1, caractérisé en ce que ce procédé est réalisé de façon continue dans une colonne.

3. Procédé selon la revendication 1 ou 2, caractérisé en ce que le métal de transition est le cuivre, le nickel, le zinc ou le cobalt.

4. Procédé selon l'une quelconque des revendications 1 à 3, caractérisé en ce que dans l'étape c) les protéines sont désorbées au moyen d'eau distillée ou en augmentant le pH à une valeur de 8-10, après quoi tout métal de transition éventuellement présent est enlevé par une substance chélatée.

5. Procédé selon l'une quelconque des revendications 1 à 4, caractérisé en ce que dans l'étape c) un sel est ajouté jusqu'à un maximum de 1 M au cours de la désorption.

6. Procédé selon l'une quelconque des revendications 1 à 5, caractérisé en ce que les protéines désorbées consistent principalement en immunoglobulines, lactoferrine et lactoperoxydase.

7. Procédé de traitement du lait, caractérisé en ce qu'on soumet du lait non stérilisé écrémé à une chromatographie sur colonne avec interaction de chélate métallique, comprenant les étapes consécutives de:
a) mise en contact du lait avec un métal de transition qui est chélaté à une matrice fonctionnelle ayant une résistance mécanique et une porosité telles que pour une colonne ayant une longueur de 30 cm et lorsqu'on utilise un débit linéaire de 150 cm/h, la pression est dans un intervalle de 0-5 bar et est stable, à la suite de quoi les protéines du lait sont liées à la matrice,
b) enlèvement de la fraction centenant les composants du lait non liés à la matrice,
c) désorption des protéines liées à la matrice, et d) mise en contact de la fraction obtenue dans l'étape b) au moins une fois avec une matrice fonctionnelle telle qu'utilisée dans l'étape a) , mais cette fois non chargée en métal de transition, ce qui fait que le métal présent dans la fraction est complètement ou substantiellement enlevé.

8. Procédé selon la revendication 7, caractérisé en ce que la fraction obtenue dans l'étape b) est mise en contact 3 à 7 fois de façon discontinue avec à chaque fois une matrice fonctionnelle fraîche dans l'étape d).

9. Procédé selon la revendication 7 ou 8, caractérisé en ce que la fraction obtenue dans l'étape d) est ensuite stérilisée, après quoi les protéines obtenues dans l'étape c) y sont ajoutées.
